# EUROPEAN PATENT APPLICATION

(11) **EP 4 039 238 A1**
(43) Date of publication of application: **10.08.2022**
(21) Application number: 20881954.0
(22) Date of filing: 27.10.2020
(51) Int. Cl.: A61F 13/49, A61F 13/532

(54) **ABSORBER AND DISPOSABLE DIAPER EQUIPPED WITH SAME**

(30) Priority: 28.10.2019 JP 2019194942
(71) Applicant: Zuiko Corporation, Settsu-shi Osaka 566-0045 (JP)
(72) Inventor: FURUKAWA, Daisuke, Settsu-shi, Osaka 566-0045 (JP); SHIMADA, Takahiro, Settsu-shi, Osaka 566-0045 (JP)
(74) Representative: Müller-Boré & Partner Patentanwälte PartG mbB
(86) International application number: PCT/JP2020/040164
(87) International publication number: WO 2021/085399

(57) **Abstract**

The present invention aims to achieve both a function of diffusing excretions or the like in a longitudinal direction of an absorber and maintenance of adhesion to a wearer in a direction orthogonal to the longitudinal direction. The absorber includes a top sheet (21), a back sheet (22) opposed to the top sheet (21), and an absorbent core (23) extending in a predetermined longitudinal direction between the top sheet (21) and the back sheet (22) and configured to absorb excretions or the like of the wearer. The absorbent core (23) has a plurality of opening portions (23a) each having a shape that opens toward the top sheet (21) and extends in the longitudinal direction and each arranged in the longitudinal direction. An interval (G1) in the longitudinal direction between two opening portions (23a) adjacent to each other among the plurality of opening portions (23a) is set to a dimension equal to or larger than a dimension (D1) in the longitudinal direction of each of the two adjacent opening portions (23a).

## Description

### Technical Field

The present invention relates to an absorber for absorbing excretions or the like of a wearer, and a disposable diaper including the same.

### Background Art

Conventionally, absorbers used for disposable diapers and sanitary napkins are known.

For example, an auxiliary pad described in Patent Literature 1 includes a top sheet, a back sheet opposed to the top sheet, and an absorption laminate provided between the top sheet and the back sheet.

The absorption laminate has a shape extending in a predetermined longitudinal direction. In addition, the absorption laminate includes a lower absorber provided on the back sheet and an upper absorber provided between the lower absorber and the top sheet.

In addition, the absorption laminate has a configuration for enhancing adhesion to a wearer even when the auxiliary pad is twisted in accordance with movement of the wearer. Specifically, a rectangular opening portion continuously extending in a longitudinal direction of the absorption laminate (a longitudinal direction of the upper absorber) is formed in the upper absorber. This causes the opening portion to be closed when the auxiliary pad is about to be twisted, thereby enabling an upper surface of the upper absorber 31 to be maintained flat, so that adhesion to the wearer can be enhanced.

Furthermore, since the opening portion continuously extends in the longitudinal direction of the upper absorber, excretions or the like can be diffused in the longitudinal direction, and the excretions or the like can be efficiently absorbed as compared with a case where the opening portion is not formed.

As described above, although it is preferable that the opening portion is formed to be relatively long in the longitudinal direction in order to diffuse excretions or the like in the longitudinal direction, in a case where the opening portion is too long, there occurs a problem that a bending strength of the absorbent pad in a direction orthogonal to the longitudinal direction decreases to lower adhesion to a wearer in the same direction.

### Citation List

### Patent Literature

Patent Literature 1: JP 2007-202575 A

### Summary of Invention

The present invention has been made in view of the above problem, and an object of the present invention is to provide an absorber capable of achieving both a function of diffusing excretions or the like in a longitudinal direction of the absorber and maintenance of adhesion to a wearer in a direction orthogonal to the longitudinal direction, and a disposable diaper including the absorber.

In order to solve the above problem, the present invention provides an absorber for absorbing excretions or the like of a wearer, the absorber including: a top sheet; a back sheet opposed to the top sheet; and an absorbent core extending in a predetermined longitudinal direction between the top sheet and the back sheet and configured to absorb excretions or the like of a wearer. The absorbent core has a plurality of opening portions each having a shape that opens toward the top sheet and extends in the longitudinal direction and each arranged in the longitudinal direction, and an interval in the longitudinal direction between two opening portions adjacent to each other among the plurality of opening portions is set to a dimension equal to or larger than a dimension in the longitudinal direction of each of the two adjacent opening portions.

Furthermore, the present invention provides a disposable diaper including: a front abdomen portion for covering an abdomen of a wearer; a back portion for covering buttocks of the wearer; and the absorber extending between the front abdomen portion and the back portion via a crotch portion of the wearer, in which a portion having one of the non-formation regions in the absorber is connected to the front abdomen portion, and a portion having the other of the non-formation regions in the absorber is connected to the back portion.

According to the present invention, it is possible to achieve both a function of diffusing excretions or the like in a longitudinal direction of an absorber and maintenance of adhesion to a wearer in a direction orthogonal to the longitudinal direction.

### Brief Description of Drawings

FIG. 1 is a perspective view illustrating an overall configuration of a disposable diaper according to an embodiment of the present invention.
FIG. 2 is a developed view of the disposable diaper shown in FIG. 1.
FIG. 3 is an exploded perspective view of the disposable diaper shown in FIG. 2.
FIG. 4 is a cross-sectional view taken along line IV-IV in FIG. 2.
FIG. 5 is a plan view illustrating an absorbent core shown in FIG. 4.
FIG. 6 is a plan view of an absorbent core according to another embodiment of the present invention.
FIG. 7 is a perspective view illustrating an overall configuration of a disposable diaper according to the other embodiment of the present invention.

### Description of Embodiments

In the following, embodiments of the present invention will be described with reference to the accompanying drawings. Note that the following embodiments are examples embodying the present invention and do not limit the technical scope of the present invention.

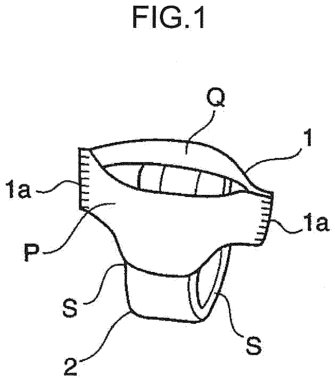

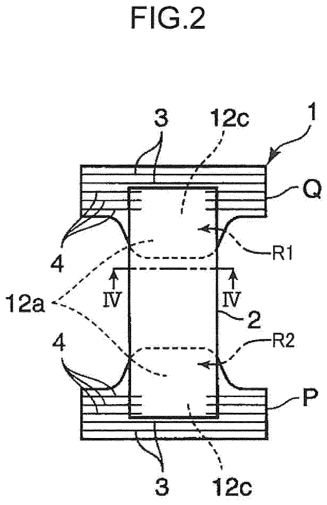

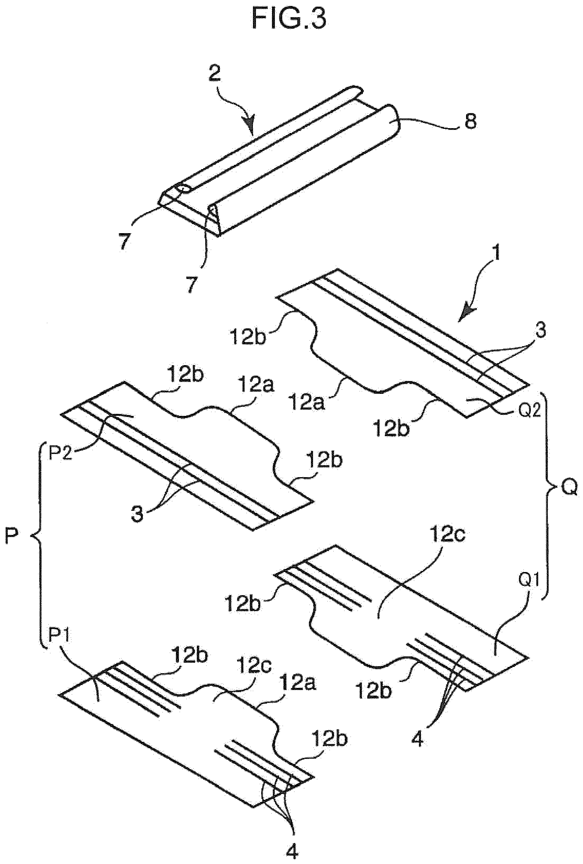

FIG. 1 is a perspective view illustrating an overall configuration of a disposable diaper 1 according to an embodiment of the present invention. FIG. 2 is a developed view of the disposable diaper 1 shown in FIG. 1. FIG. 3 is an exploded perspective view of the disposable diaper shown in FIG. 2.

With reference to FIG. I to FIG. 3, the disposable diaper 1 includes a front abdomen portion P for covering an abdomen of a wearer, a back portion Q for covering buttocks of the wearer, and an absorber 2 provided between the front abdomen portion P and the back portion Q via a crotch portion of the wearer.

One end portion of the absorber 2 in a longitudinal direction is connected to the front abdomen portion P, and the other end portion of the absorber 2 in the longitudinal direction is connected to the back portion Q. In a state where such absorber 2 as connected to the front abdomen portion P and the back portion Q is folded in two, both side portions 1a of the front abdomen portion P and the back portion Q opposed to each other are side sealed (hereinafter referred to as "side seal 1a"). As a result, leg opening portions S are formed on both sides of the absorber 2.

As illustrated in FIG. 2 and FIG. 3, the front abdomen portion P includes an inner sheet P2 disposed on a skin surface side of the wearer, an outer sheet P1 disposed on a side opposite to the skin surface of the wearer with respect to the inner sheet P2, a waist elastic member 3 fixed to the inner sheet P2, and a body fitting elastic member 4 fixed to the inner sheet P2 and the outer sheet P1.

The inner sheet P2 and the outer sheet P1 (which will be hereinafter referred to as both the sheets P1 and P2 unless otherwise distinction is required) are made of nonwoven fabric and have the same shape. Specifically, both the sheets P1 and P2 have a planar shape including a protrusion 12a and a pair of recesses 12b provided on both sides of the protrusion 12a.

The back portion Q includes an inner sheet Q2 disposed on the skin surface side of the wearer, an outer sheet Q1 disposed on a side opposite to the skin surface of the wearer with respect to the inner sheet Q2, the waist elastic member 3 fixed to the inner sheet Q2, and the body fitting elastic member 4 fixed to the inner sheet Q2 and the outer sheet Q1.

The inner sheet Q2 and the outer sheet Q1 (which will be hereinafter referred to as both the sheets Q1 and Q2 unless otherwise distinction is required) are made of nonwoven fabric and have the same shape. Specifically, both the sheets Q1 and Q2 have a planar shape including the protrusion 12a and the pair of recesses 12b provided on both sides of the protrusion 12a.

The waist elastic member 3 is bonded to the inner sheets P2 and Q2 in a state of being extended in a longitudinal direction (a left-right direction in FIG. 2) of the inner sheets P2 and Q2.

The body fitting elastic member 4 is sandwiched between both the sheets P1 and P2, and is bonded to both the sheets P1 and P2 in a state of being extended in the longitudinal direction (the left-right direction in FIG. 2) of the sheets P1 and P2. Further, the body fitting elastic member 4 is sandwiched between both the sheets Q1 and Q2, and is bonded to both the sheets Q1 and Q2 in a state of being extended in the longitudinal direction of the sheets Q1 and Q2.

The body fitting elastic member 4 is cut within a range in which the protrusion 12a is formed in the longitudinal direction (the left-right direction in FIG. 2) of the front abdomen portion P and the back portion Q. As a result, a weakened portion 12c in which contraction force of the body fitting elastic member 4 is reduced is formed in each of the front abdomen portion P and the back portion Q.

As illustrated in FIG. 2, the absorber 2 is connected to the front abdomen portion P and the back portion Q so as to extend over the front abdomen portion P and the back portion Q. Specifically, the one end portion of the absorber 2 in the longitudinal direction (an up-down direction in FIG. 2) is bonded to the back portion Q in a state of overlapping the protrusion 12a and the weakened portion 12c in the back portion Q. In addition, the other end portion of the absorber 2 in the longitudinal direction is bonded to the front abdomen portion P in a state of overlapping the protrusion 12a and the weakened portion 12c in the front abdomen portion P.

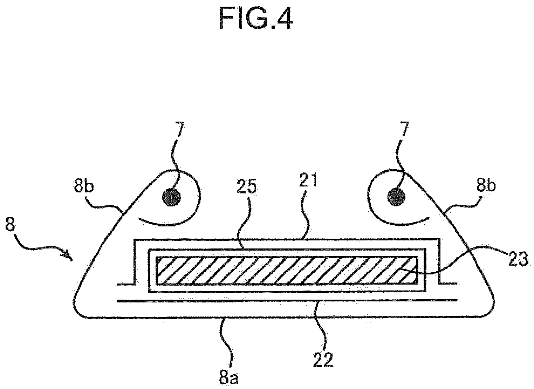

FIG. 4 is a cross-sectional view illustrating a state in which the absorber 2 in FIG. 2 is cut in a direction orthogonal to the longitudinal direction, that is, a cross-sectional view taken along line IV-IV in FIG. 2.

The absorber 2 includes a top sheet 21, a back sheet 22 opposed to the top sheet 21, an absorbent core 23 provided between the top sheet 21 and the back sheet 22, a tissue 25 enclosing the absorbent core 23, and a rising flap 8 that regulates movement of excretions or the like to the side of the absorbent core 23.

The top sheet 21 is made of a material (nonwoven fabric in the present embodiment) having a liquid permeation property.

The tissue 25 encloses the absorbent core 23 in order to maintain a shape of the absorbent core 23 between the top sheet 21 and the back sheet 22. The tissue 25 is made of a material that allows liquid to permeate therethrough.

The back sheet 22 is made of a material (in the present embodiment, polyethylene or nonwoven fabric having water repellency and air permeability) that regulates liquid permeation.

The top sheet 21 and the back sheet 22 are in close contact with each other over the entire periphery of the absorbent core 23, and both the sheets 21 and 22 are bonded to each other at close contact positions. The absorbent core 23 is also bonded to the back sheet 22.

The rising flap 8 includes a base portion 8a bonded to a lower surface of the back sheet 22, a pair of rising portions 8b extending from both side positions of the absorbent core 23 in the base portion 8a to a position above the absorbent core 23, and an elastic member 7 provided at a distal end portion of each of the rising portions 8b. The base portion 8a and the rising portion 8b are made of a material (in the present embodiment, polyethylene or nonwoven fabric having water repellency and air permeability) that regulates liquid permeation. In a case where the base portion 8a and the rising portion 8b are made of a material that regulates liquid permeation as described above, the back sheet 22 may be made of a material having a liquid permeation property.

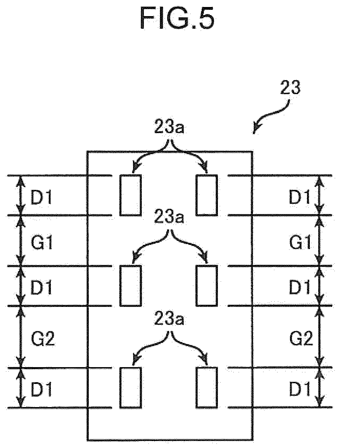

FIG. 5 is a plan view illustrating the absorbent core 23 of FIG. 4.

With reference to FIG. 5, the absorbent core 23 extends in the longitudinal direction (the up-down direction in FIG. 2, the same applies hereinafter) of the absorber 2 and absorbs excretions or the like of the wearer. Specifically, the absorbent core 23 is obtained by laminating (molding) pulverized pulp (fluff). The absorbent core 23 may contain a super absorbent polymer in addition to the fluff.

The absorbent core 23 has a plurality of opening portions 23a each having a shape that opens toward the top sheet 21 and extends in the longitudinal direction, and each being arranged in the longitudinal direction. In the absorbent core 23 of the present embodiment, there are formed two rows of three opening portions 23a arranged in the longitudinal direction. Although the three opening portions 23a in each row are arranged at the same position in the longitudinal direction, the opening portions may be arranged at different positions in the longitudinal direction.

The opening portion 23a extends so as to penetrate from a surface of the absorbent core 23 on the top sheet 21 side to a surface of the absorbent core 23 on the back sheet 22 side. The opening portion 23a does not need to penetrate the absorbent core 23, and an opening portion 23a having a bottom on the back sheet 22 side can be also formed.

In addition, an interval G1, G2 in a longitudinal direction between two adjacent opening portions among the plurality of opening portions 23a is set to a dimension equal to or larger than a dimension D1 in the longitudinal direction of each of the two adjacent opening portions 23a.

Specifically, the longitudinal dimensions D1 of all the opening portions 23a are set to substantially the same dimension. Further, regarding the intervals G1 and G2 in the longitudinal direction between the two opening portions 23a adjacent to each other in each row, the interval G2 is set to be larger than the interval G1. Then, the interval G1 is set to be equal to or larger than the dimension D1. Although the example in which the interval G2 is set to be larger than the interval G1 has been described, all the intervals may be set to substantially the same dimension.

As described above, according to the above embodiment, since there are provided the plurality of opening portions 23a having the shape extending in the longitudinal direction and arranged in the longitudinal direction, excretions or the like of the wearer can be diffused in the longitudinal direction through these opening portions 23a. Moreover, since the interval G1, G2 between the two opening portions 23a adjacent to each other is set to be equal to or larger than the dimension D1 of the opening portion 23a in the longitudinal direction, the interval G1, G2 between the opening portions 23a, that is, a region where the opening portion 23a is not formed, can be secured to be relatively wide, and a bending strength of the absorbent core 23 in a direction orthogonal to the longitudinal direction can be maintained. Therefore, as described above, it is possible to maintain the adhesion of the absorbent core 23 to the wearer while securing the function of diffusing excretions or the like in the longitudinal direction by the plurality of opening portions 23a.

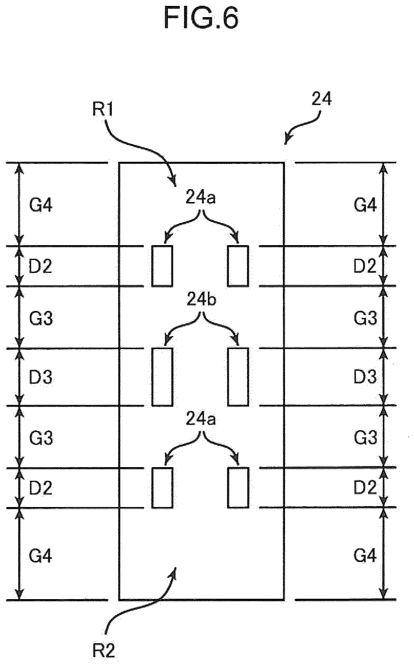

Although in the above embodiment, the dimension D1 of the opening portion in the longitudinal direction is set to substantially the same dimension, the dimension of the opening portion in the longitudinal direction may be set to a different value for each opening portion as in an absorbent core 24 illustrated in FIG. 6.

FIG. 6 is a plan view of the absorbent core 24 according to another embodiment of the present invention. In the following, portions of the absorbent core 24 different from those of the absorbent core 23 illustrated in FIG. 5 will be mainly described with reference to FIG. 6.

In the absorbent core 24, two rows of three opening portions 24a and 24b arranged in the longitudinal direction are formed. Since a configuration and arrangement of the opening portions 24a and 24b in each row are common, the right row will be described.

The right row includes the two opening portions 24a having a longitudinal dimension D2 and one opening portion 24b provided between the opening portions 24a and having a longitudinal dimension D3 longer than the dimension D2.

Intervals G3 between the opening portion 24b and both the opening portions 24a are set to be substantially the same, and are set to be equal to or larger than the largest dimension D3 among the dimensions D2 and D3 in the longitudinal direction of the opening portions 24a and 24b. The two intervals G3 can be set to different dimensions on condition that the two intervals G3 are equal to or larger than the dimension D3.

As a result, unlike the case where the interval between the opening portions 24a and 24b adjacent to each other is managed on the basis of the two dimensions of the opening portions adjacent to each other, it is possible to efficiently manage all the intervals between the opening portions 24a and 24b on the basis of one criterion, i.e., the dimension D3 of the longest opening portion 24b in the longitudinal direction.

Further, non-formation regions R1 and R2, where the opening portions 24a and 24b are not formed, are formed at both end portions of the absorbent core 24 of the present embodiment similarly to the above embodiment, and a dimension G4 of the non-formation regions R1 and R2 in a longitudinal direction is set to be equal to or larger than the dimension D3. This enables the absorber 2 to secure a sufficient strength at both ends of the absorbent core 24.

Therefore, as illustrated in FIG. 2, use of the non-formation regions R1 and R2 as connection portions with the front abdomen portion P and the back portion Q secures sufficient strength of the connection portions. In other words, in the disposable diaper 1 to which the absorbent core 24 of the present embodiment is applied, it is possible to adopt a configuration in which a portion having the non-formation region R1 in the absorber 2 is connected to the front abdomen portion P, and a portion having the non-formation region R2 in the absorber 2 is connected to the back portion Q.

According to such disposable diaper 1, since the absorber 2 can be connected to the front abdomen portion P and the back portion Q in the non-formation regions R1 and R2 where the strength is secured as described above, the connection strength between the absorber and the front abdomen portion P and the back portion Q can be sufficiently secured.

Although in the above embodiment, the dimensions G4 in the longitudinal direction of the non-formation regions R1 and R2 are set to be substantially the same, the dimension in the longitudinal direction of each of the non-formation regions R1 and R2 may be individually set.

In a case where the opening portions 23a, 24a, and 24b are formed so as to penetrate the absorbent cores 23 and 24 as in the above embodiments, the top sheet 21 and the back sheet 22 may be bonded to each other through the opening portions 23a, 24a, and 24b. In this manner, the movement of the absorbent cores 23 and 24 with respect to both the sheets 21 and 22 can be reliably regulated.

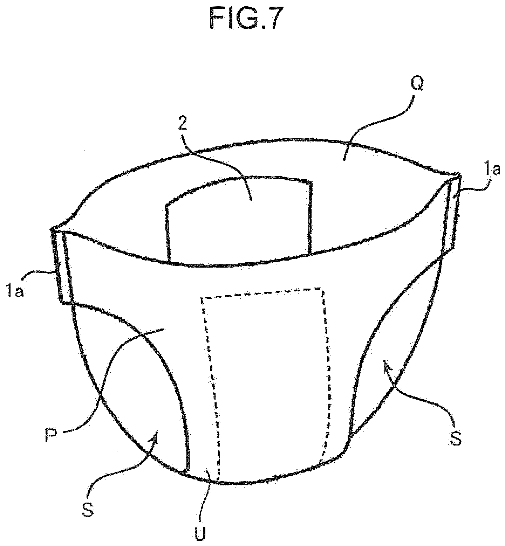

Furthermore, although in the above embodiment, the description has been made of the disposable diaper 1 in which the absorber 2 is provided so as to extend over the front abdomen portion P and the back portion Q, the absorber 2 may be provided on a sheet including the front abdomen portion P, the back portion Q, and a crotch portion U so as to cover the crotch portion U as illustrated in FIG. 7.

As the elastic members 3, 4, and 7 in the above-described embodiment, those containing polyurethane, natural rubber, or thermoplastic resin can be adopted. In addition, as the shape of the elastic members 3, 4, and 7, a thread-like shape or a ribbon-like shape can be adopted. Furthermore, in a case of adopting the thermoplastic elastic members 3, 4, and 7, an adhesive can be omitted when the members themselves have a property enabling bonding to the sheets P1, P2, Q1, and Q2.

Furthermore, in the above-described embodiments, as illustrated in FIG. 1 and FIG. 7, the example has been described in which the absorber 2 is applied to an underpants-type disposable diaper in which the front abdomen portion P and the back portion Q are bonded by the side seal 1a. However, the disposable diaper as an application target is not limited to an underpants-type disposable diaper, and by omitting the side seal 1a and adding a surface tape, the absorber 2 can be applied also to a tape-type disposable diaper from which the front abdomen portion P and the back portion Q are detachable.

Note that the above-described specific embodiments mainly include inventions having the following configurations.

Specifically, the present invention provides an absorber for absorbing excretions or the like of a wearer, the absorber including: a top sheet; a back sheet opposed to the top sheet; and an absorbent core extending in a predetermined longitudinal direction between the top sheet and the back sheet and configured to absorb excretions or the like of a wearer. The absorbent core has a plurality of opening portions each having a shape that opens toward the top sheet and extends in the longitudinal direction and each arranged in the longitudinal direction, and an interval in the longitudinal direction between two opening portions adjacent to each other among the plurality of opening portions is set to a dimension equal to or larger than a dimension in the longitudinal direction of each of the two adjacent opening portions.

According to the present invention, since there are provided the plurality of opening portions having the shape extending in the longitudinal direction and arranged in the longitudinal direction, excretions or the like of a wearer can be diffused in the longitudinal direction through these opening portions. Moreover, since the interval between two opening portions adjacent to each other is set to be equal to or larger than the dimensions of the opening portions in the longitudinal direction, it is possible to secure a relatively wide interval between the opening portions, that is, a relatively wide region where no opening portion is formed, and to maintain a bending strength of the absorbent core in a direction orthogonal to the longitudinal direction. It is accordingly possible to maintain the adhesion of the absorbent core to the wearer while securing the function of diffusing excretions or the like in the longitudinal direction by the plurality of opening portions as described above.

Here, in a case where the dimensions in the longitudinal direction of the plurality of opening portions are set to different dimensions, while the above described effect can be obtained by individually setting the interval between the adjacent opening portions on the basis of the two dimensions of the adjacent opening portions, it is troublesome to manage a dimension differing for each interval.

It is therefore preferable that in the absorber, all of intervals in the longitudinal direction between two opening portions adjacent to each other among the plurality of opening portions are set to be equal to or larger than a largest dimension among dimensions in the longitudinal direction of the plurality of opening portions.

According to this aspect, all the dimensions of the intervals between the opening portions can be efficiently managed on the basis of one criterion, i.e., a longitudinal dimension of a longest opening portion.

For example, while in a case of bonding an absorber onto a sheet (crotch portion) for forming a front abdomen portion, a back portion, and the crotch portion, the absorber is required to have no great bending strength, in a case where the absorber is bonded to extend over the front abdomen portion and the back portion, both end portions of the absorber are required to have a certain degree of strength.

It is therefore preferable that in the absorber, non-formation regions where the plurality of opening portions are not formed are provided at both ends of the absorbent core in the longitudinal direction, respectively, and a dimension in the longitudinal direction of each of both the non-formation regions is set to be equal to or larger than a largest dimension among dimensions in the longitudinal direction of the plurality of opening portions.

According to this aspect, since the non-formation regions in which the opening portion is not formed are provided at both ends in the longitudinal direction of the absorbent core, a sufficient strength of the absorber can be secured at both ends of the absorbent core.

Furthermore, the present invention provides a disposable diaper including: a front abdomen portion for covering an abdomen of a wearer; a back portion for covering buttocks of the wearer; and the absorber extending between the front abdomen portion and the back portion via a crotch portion of the wearer, in which a portion having one of the non-formation regions in the absorber is connected to the front abdomen portion, and a portion having the other of the non-formation regions in the absorber is connected to the back portion.

According to the present invention, since the absorber can be connected to the front abdomen portion and the back portion in the non-formation region where the strength is secured as described above, a connection strength between the absorber and the front abdomen portion and the back portion can be sufficiently secured.

In addition, as described above, since the absorber has a plurality of opening portions having the shape extending in the longitudinal direction and arranged in the longitudinal direction, excretions or the like of the wearer can be diffused in the longitudinal direction through these opening portions. Moreover, since the interval between two opening portions adjacent to each other is set to be equal to or larger than the dimensions of the opening portions in the longitudinal direction, it is possible to secure a relatively wide interval between the opening portions, that is, a relatively wide region where no opening portion is formed, and to maintain a bending strength of the absorbent core in a direction orthogonal to the longitudinal direction. It is accordingly possible to maintain the adhesion of the absorbent core to the wearer while securing the function of diffusing excretions or the like in the longitudinal direction by the plurality of opening portions as described above.

## Claims

1. An absorber for absorbing excretions or the like of a wearer, the absorber comprising:
a top sheet;
a back sheet opposed to the top sheet; and
an absorbent core extending in a predetermined longitudinal direction between the top sheet and the back sheet and configured to absorb excretions or the like of a wearer,
wherein the absorbent core has a plurality of opening portions each having a shape that opens toward the top sheet and extends in the longitudinal direction and each arranged in the longitudinal direction, and
an interval in the longitudinal direction between two opening portions adjacent to each other among the plurality of opening portions is set to a dimension equal to or larger than a dimension in the longitudinal direction of each of the two adjacent opening portions.

2. The absorber according to claim 1, wherein all of intervals in the longitudinal direction between two opening portions adjacent to each other among the plurality of opening portions are set to be equal to or larger than a largest dimension among dimensions in the longitudinal direction of the plurality of opening portions.

3. The absorber according to claim 1 or 2, wherein
non-formation regions where the plurality of opening portions are not formed are provided at both ends of the absorbent core in the longitudinal direction, respectively, and
a dimension in the longitudinal direction of each of both the non-formation regions is set to be equal to or larger than a largest dimension among dimensions in the longitudinal direction of the plurality of opening portions.

4. A disposable diaper comprising:
a front abdomen portion for covering an abdomen of a wearer;
a back portion for covering buttocks of the wearer; and
the absorber according to claim 3, the absorber extending between the front abdomen portion and the back portion via a crotch portion of the wearer,
wherein a portion having one of the non-formation regions in the absorber is connected to the front abdomen portion, and a portion having the other of the non-formation regions in the absorber is connected to the back portion.
